# EUROPÄISCHE PATENTSCHRIFT

(11) **EP 0 815 050 B1**
(45) Veröffentlichungstag und Bekanntmachung des Hinweises auf die Patenterteilung: **04.11.1998**
(21) Anmeldenummer: 96907254.5
(22) Anmeldetag: 18.03.1996
(51) Int. Cl.: C01B 13/00, H01T 23/00, A61M 15/02

(54) **VERFAHREN UND VORRICHTUNG ZUR ERZEUGUNG VON GASFÖRMIGEN SAUERSTOFFANIONENRADIKALEN**
PROCESS AND DEVICE FOR GENERATING GASEOUS OXYGEN ANIONIC RADICALS
PROCEDE ET DISPOSITIF DE PRODUCTION DE RADICAUX ANIONIQUES D'OXYGENE GAZEUX

(30) Priorität: 24.03.1995 DE 19512228
(43) Veröffentlichungstag der Anmeldung: 07.01.1998
(73) Patentinhaber: Goldstein & Lewin Technology GmbH, 14532 Stahnsdorf (DE)
(72) Erfinder: GOLDSTEIN, Naum, D-10785 Berlin (DE); LEWIN, Thomas, D-10779 Berlin (DE)
(74) Vertreter: Wablat, Wolfgang, Dr.Dr.
(86) Internationale Anmeldenummer: DE9600492
(87) Internationale Veröffentlichungsnummer: WO9630296

(56) Entgegenhaltungen:
- EP-A- 0 465 783
- DE-A- 3 411 335
- DE-A- 3 838 210
- DE-A- 4 112 459
- DE-A- 4 234 707
- FR-A- 2 571 554

## Beschreibung

Die Erfindung betrifft ein Verfahren und eine Vorrichtung zur Erzeugung von gasförmigen Sauerstoffanionenradikalen unter atmosphärischen Druckbedingungen mit einer aus Kohlenstoffasern bestehenden Ionisationselektrode.

In der DE 42 34 707 A1 ist bereits eine gattungsgemäße Vorrichtung beschrieben, bei der medizinischer Sauerstoff ionisiert wird, um gegenüber herkömmlichen Luftionisationsgeräten eine höhere Konzentration an Superoxid zu erzielen und die Bildung von Stickoxiden sowie Ozon zu vermeiden.

Die Ionisierung von medizinischem Sauerstoff ist insoweit vorteilhaft gegenüber der Ionisierung von Luft. Die Bereitstellung von medizinischem Sauerstoff ist jedoch mit einem größeren apparativen Aufwand und zusätzlichen Kosten verbunden. Darüberhinaus kann es auch bei der Ionisierung von medizinischem Sauerstoff zur Bildung schädlicher Nebenprodukte kommen, und zwar in Form von Ozon.

Der vorliegenden Erfindung liegt daher die Aufgabe zugrunde, ein Verfahren und eine Vorrichtung zur kostengünstigen Erzeugung von Sauerstoffanionenradikalen zu schaffen, die insbesondere bei einer Ionisierung von Luft eine schädigende Belastung durch unerwünschte Nebenprodukte des Ionisationsprozesses ausschließen.

Diese Aufgabe wird hinsichtlich des erfindungsgemäßen Verfahrens dadurch gelöst, daß ungeladene Nebenprodukte des Ionisationsprozesses, wie Stickoxide und Ozon, von den erzeugten Sauerstoffanionenradikalen durch Absaugen abgetrennt werden.

Die erfindungsgemäße Lösung verhindert eine schädigende Belastung der zu behandelnden Personen durch gesundheitsschädliche Nebenprodukte des Ionisationsprozesses, die insbesondere bei der Ionisierung von Luft in Form von Stickoxiden und Ozon entstehen können.

Eine optimale Abtrennung ungeladener Nebenprodukte des Ionisationsprozesses wird nach einer bevorzugten Ausgestaltung der erfindungsgemäßen Lösung dadurch erreicht, daß im Bereich der Ionisationselektrode eine gerichtete Saugströmung eingestellt wird, deren Saugwirkung im wesentlichen auf den engeren Bereich der Ionisationselektrode begrenzt ist und die erzeugten Sauerstoffanionenradikale an einem davon beabstandeten Applikationsort im wesentlichen nicht erfaßt.

Eine andere bevorzugte Ausgestaltung der erfindungsgemäßen Lösung besteht darin, daß an der Ionisationselektrode eine negative Spannung von -1 bis -7 KV zum Bezugspotential einer Hochspannungsquelle angeschlossen ist. Erst ab einer negativen Spannung von -5 KV konnte eine signifikante Ozonbildung festgestellt werden, der jedoch erfindungsgemäß durch eine höhere Absaugleistung entgegengewirkt werden kann. Auf diese Weise kann entweder die Bildung von Ozon gänzlich vermieden oder zumindest eine schädigende Wirkung ausgeschlossen werden.

Bei Versuchen mit einem erfindungsgemäßen Prototyp ist an dessen Ionisationselektrode eine negative Hochspannung zwischen -3 bis -4 KV erzeugt worden. Ausgehend von dem daraus resultierenden Potentialfeld und der elektrischen Feldstärkeverteilung um die Ionisationselektrode sowie der hochohmigen Anpassung (1 Gigaohm) der Gegenelektrode an das Bezugspotential der Schaltung konnten ein Potentialunterschied von 2 KV zwischen den Elektroden und ein Ruhestrom von 1 µA gemessen werden. In einem Abstand von 2 cm zur Ionisationselektrode wurden Ozonkonzentrationen gemessen, die unterhalb bzw. innerhalb der üblichen Ozonkonzentrationen der Umgebungsluft liegen.

Eine weitere bevorzugte Ausgestaltung der erfindungsgemäßen Lösung besteht darin, daß das zu ionisierende Gas, die Ionisationselektrode und/oder die Umgebung der Ionisationselektrode beheizt werden. Hierdurch kann eine frühzeitige Entladung der erzeugten Sauerstoffanionenradikale verhindert werden, indem der Feuchtegehalt des zu ionisierenden Gases bzw. der Luft verringert wird. Zudem wird hinsichtlich der Atemluft eine Kondensatbildung an den Elektroden verhindert.

Da die Menge der erzeugten Sauerstoffanionenradikale andererseits von dem Bereich der angelegten Hochspannung abhängt, mit hoher Spannung aber die Ozonbildung zunimmt, ist die Erzeugung einer ausreichend hohen Menge an Sauerstoffanionenradikalen ohne signifikante Ozonbildung durch Anlegen einer relativ geringen Hochspannung möglich.

Weitere vorteilhafte Ausgestaltungen der Erfindung sind in den Unteransprüchen gekennzeichnet.

Im folgenden wird die Erfindung anhand verschiedener Ausführungsbeispiele und mit Bezug auf die Figuren der beiliegenden Zeichnung näher erläutert. Es zeigen:
- Fig. 1: eine Seitenansicht einer im Längsschnitt dargestellten erfindungsgemäßen Vorrichtung in Form eines Inhalationsgerätes zur Behandlung von Atemwegserkrankungen mit einem Abstandhalter in geöffneter Stellung;
- Fig. 2: eine Vorderansicht des in Fig.1 dargestellten Inhalationsgerätes mit geschlossenem Abstandhalter;
- Fig. 3: eine Seitenansicht einer teilweise geschnitten dargestellten Ionisationselektrode mit Gegenelektrode und Reflektor;
- Fig. 4: einen Ausschnitt des Inhalationsgerätes mit einer Draufsicht auf die Ionisationselektrode, die Gegenelektrode und den Reflektor gemäß Fig. 3;
- Fig. 5: eine Seitenansicht eines anderen Ausführungsbeispiels mit einer teilweise geschnitten dargestellten Ionisationselektrode, einer Gegenelektrode und einem Reflektor;
- Fig. 6: eine Draufsicht auf die Ionisationselektrode, die Gegenelektrode und den Reflektor gemäß Fig. 5;
- Fig. 7: eine Seitenansicht eines weiteren Ausführungsbeispiels mit einer teilweise geschnitten dargestellten Ionisationselektrode, einer Gegenelektrode und einem Reflektor; und
- Fig. 8: eine Draufsicht auf die Ionisationselektrode, die Gegenelektrode und den Reflektor gemäß Fig. 7.

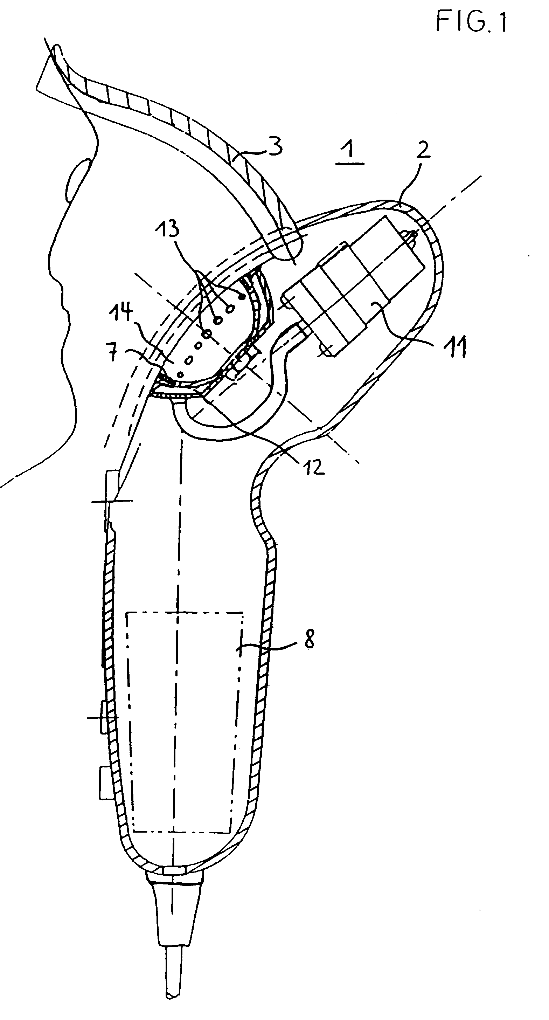

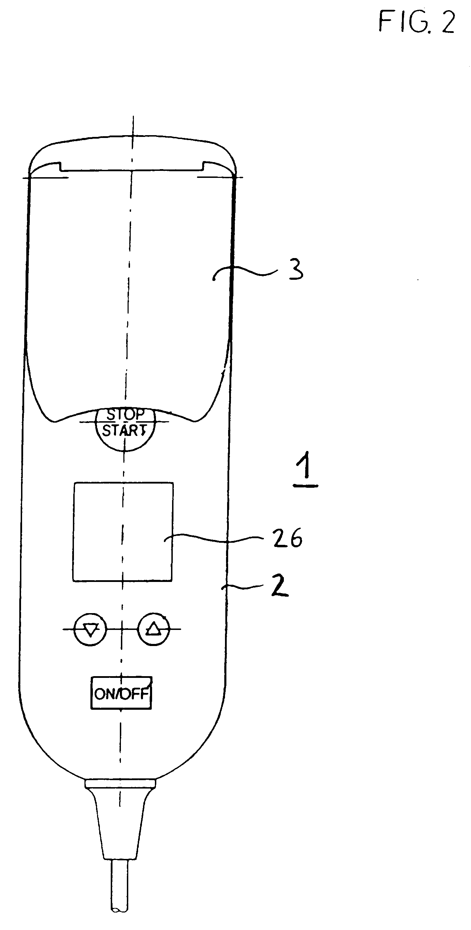

Das in den Figuren 1 und 2 schematisch dargestellte Inhalationsgerät 1 weist ein gekrümmt ausgebildetes, zweiteiliges Gehäuse 2 auf, an dem ein aufklappbarer Abstandhalter 3 angelenkt ist. Zur Inhalation der mit dem Gerät erzeugten Sauerstoffanionenradikale wird der Abstandhalter 3 aufgeklappt und an das Nasenbein der inhalierenden Person angelegt. Auf diese Weise wird sichergestellt, daß eine ausreichende Menge an Sauerstoffanionenradikalen den Applikationsort der inhalierenden Person erreicht. Der Abstand zwischen der Ionisationselektrode und dem Applikationsort sollte 10 cm nicht überschreiten, da mit zunehmendem Abstand der Verlust der Anionenradikale erheblich zunimmt.

Die Bedienung des Inhalationsgerätes 1 erfolgt über eine Tastatur, die mit entsprechenden Kontaktflächen einer elektronischen Schaltung 8 verbunden ist. Das Bedienfeld umfaßt eine Ein/Aus-Taste, zwei Einstelltasten, mit denen sich die Anwendungsdauer in Minutenschritten einstellen läßt, und eine Taste zum Starten und Unterbrechen der Anwendung. Die eingestellte Anwendungsdauer bzw. die während der Anwendung verbleibende Restdauer wird auf einem Display 26 angezeigt (vgl. Fig. 2).

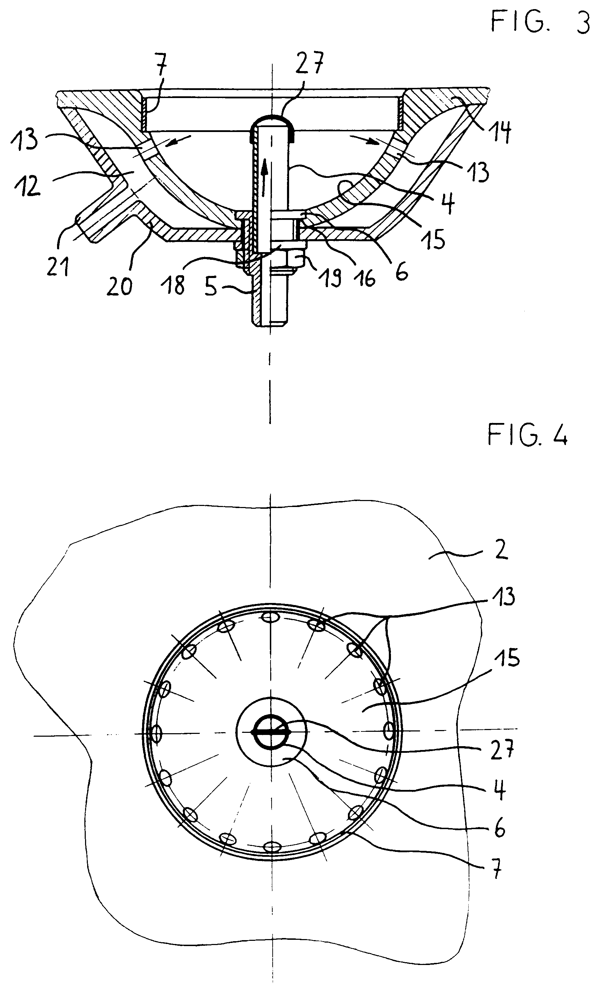

Die Erzeugung der Sauerstoffanionenradikale erfolgt mit einer aus Kohlenstoffasern 27 bestehenden Ionisationselektrode 4, die in den Figuren 3 bis 6 schematisch dargestellt ist. Die Kohlenstoffasern 27 sind zu einer Kordel zusammengefaßt, die quer über der hohlzylindrischen Elektrode 4 angeordnet ist, die ihrerseits in bzw. an einem Elektrodenhalter 5 ein- bzw. angesetzt ist.

Die Verwendung einer Kohlenstoffaser-Elektrode ist besonders vorteilhaft, da diese - im Gegensatz zu den mitunter verwendeten Metallnadel-Elektroden - keine schädlichen Metalle wie Chrom oder dergleichen freisetzt ("absputtert"). Bei der allmählichen Verbrennung der Kohlenstoffaser-Elektrode entstehen lediglich geringste Mengen Kohlendioxid.

Als zu ionisierendes Gas wird vorzugsweise Umgebungsluft verwendet, die beispielsweise über ein Miniatur-Gebläse angesaugt und über eine am hohlzylindrischen Elektrodenhalter 5 angeschlossene Leitung der Ionisationselektrode 4 zugeführt werden kann. An dieser Leitung, dem Elektrodenhalter 5 und/oder dem Reflektor 14 kann eine elektrische Heizeinrichtung angeschlossen sein, mit der sich das zu ionisierende Gas, die Ionisationselektrode 4 und/oder die Umgebung der Ionisationselektrode 4 beheizen läßt.

Die Innenbelüftung der Ionisationselektrode 4 bewirkt, daß schädliche Nebenprodukte des Ionisationsprozesses - soweit solche bei dem erfindungsgemäßen Inhalationsgerät überhaupt entstehen - so weit verdünnt werden, daß sie keine schädigende Wirkung entfalten können.

Das Inhalationsgerät 1 ist mit einer Absaugeinrichtung ausgestattet, die entsprechend dem in den Figuren 1, 3 und 4 dargestellten Ausführungsbeispiel ein Miniatur-Sauggebläse 11 und eine Saugkappe 20 mit einem Stutzen 21 umfaßt. Die Saugkappe 20 ist an der Rückseite des Reflektors 14, der mehrere Absaugöffnungen 13 aufweist, angesetzt und derart ausgebildet, daß sie mit dem Reflektor 14 eine Ringleitung 12 bildet.

Durch die periphere Anordnung der Absaugöffnungen 13 in unmittelbarer Umgebung der Ionisationselektrode 4 läßt sich eine Saugströmung einstellen, deren Saugwirkung im wesentlichen auf den engeren Bereich der Ionisationselektrode 4 begrenzt ist und die erzeugten Sauerstoffanionenradikale am Applikationsort im wesentlichen nicht erfaßt.

Im Gehäuse 2 des Inhalationsgerätes 1 ist ferner eine elektronische Schaltung 8 angeordnet, die eine relativ niedrige Spannung einer Spannungsquelle in eine ausreichend hohe negative Spannung für eine Korona-Entladung und die Erzeugung von Sauerstoffanionenradikalen an der Ionisationselektrode 4 umwandelt. Vorzugsweise beträgt die an der Ionisationselektrode 4 anliegende negative Spannung 1 bis 7 KV zum Bezugspotential der elektronischen Schaltung 8.

Der zweite Pol der negativen Hochspannung ist mit dem Patienten verbunden. Diese Verbindung wird über die Handfläche des Patienten hergestellt, der beim Festhalten des Inhalationsgerätes zwangsläufig "Erdungselektroden" berührt, welche aus einem an der Griffläche des Gerätes befestigten Blechteil sowie zwei in der Gehäuseteilung angeordneten Drahtteilen aus Aluminium bestehen.

Dient beispielsweise das 220 Volt Wechselspannungsnetz als Versorgungsquelle, so kann die elektronische Schaltung 8, welche über einen Sicherheitstrenntrafo vom Netz getrennt wird, einen Transformator und eine Kaskadenschaltung zur Spannungsversorgung der Ionisationselektrode 4 aufweisen. Zur Strombegrenzung im Kurzschlußfall (d.h. einer Berührung der Ionisationselektrode) ist eine hochohmige Ankopplung mittels Widerständen an die Kaskadenschaltung vorgesehen.

Die Ionisationselektrode 4 ist von einer ringförmigen Gegenelektrode 7 umgeben, die in einer umlaufenden Ausnehmung des Reflektors 14 angeordnet ist. Die Gegenelektrode 7 ist hochohmig, vorzugsweise mit 1 Gigaohm an das Bezugspotential der elektronischen Schaltung 8 angeschlossen. Auf diese Weise wird ein Potentialunterschied mit U_{Bezugspotential} < U_{Gegenelektrode} < U_{Ionisationselektrode} erzeugt.

Der Innendurchmesser der Gegenelektrode 7 ist so dimensioniert, daß ein bündiger Übergang zur Innenseite 15 des Reflektors 14 vorliegt. Ebenso ist in Figur 3 zu erkennen, daß der umlaufende Flansch 6 des verschraubbaren Elektrodenhalters 5 in der stufig ausgebildeten Reflektorbohrung 16 bündig versenkbar ist. Diese Maßnahmen sowie die kalottenförmige Ausbildung des Reflektors ermöglichen eine einfache Reinigung dieses Gerätebereichs.

Die Ringform der Gegenelektrode 7 sowie deren koaxiale Anordnung gegenüber der Ionisationselektrode 4 stellen sicher, daß der gesamte Bereich der Ionisationselektrode beeinflußt und der Aufbau eines hinsichtlich seiner Geometrie und Spannung definierten elektrischen Feldes ermöglicht wird. Dabei kann die Gegenelektrode 7 auch an einer Spannung kleiner 7 KV angeschlossen sein. Vorzugsweise wird im Bereich der Ionisationselektrode 4 ein elektrisches Feld zwischen 30 KV/m und 1500 KV/m eingestellt.

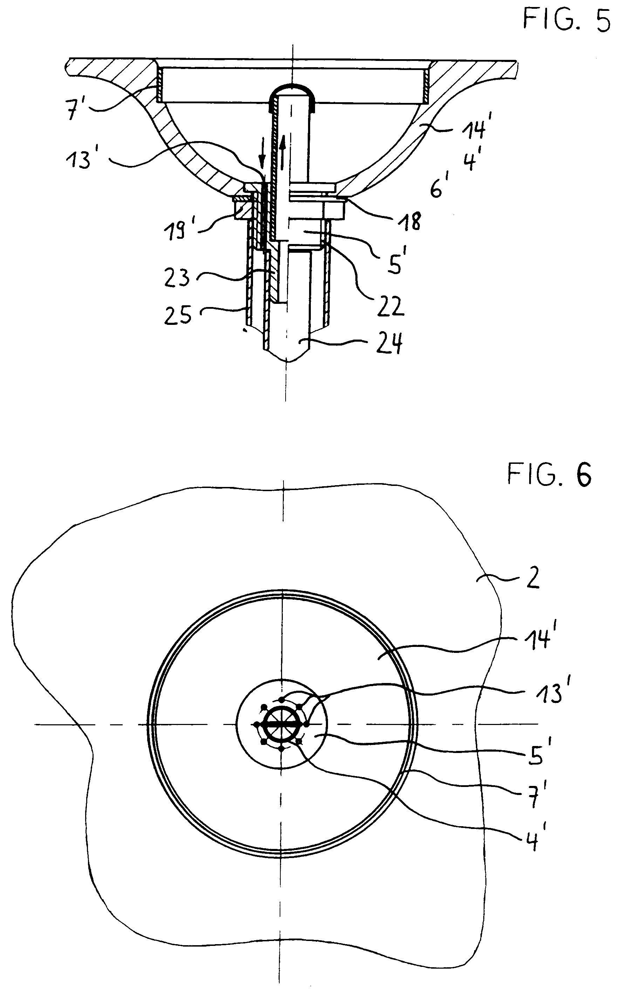

Ein weiteres Ausführungsbeispiel eines erfindungsgemäßen Inhalationsgerätes ist in den Figuren 5 und 6 gezeigt. Die Ionisationselektrode 4' ist wiederum zentrisch in einem kalottenförmigen Reflektor 14' angeordnet, und zwar mittels eines Elektrodenhalters 5', der einen im Reflektor 14 formschlüssig einsetzbaren Flansch 6' und ein im Durchmesser reduziertes Außengewinde 22 mit einer aufgeschraubten Mutter 19' aufweist. Der Elektrodenhalter 5' ist zudem mit mehreren Absaugöffnungen 13' versehen, die unmittelbar an die Ionisationselektrode 4' angrenzen. Unterhalb des Außengewindes 22 ist ein Stutzen 23 ausgebildet, der seinerseits gegenüber dem Außengewinde 22 einen reduzierten Außendurchmeser aufweist. Zur gezielten Absaugung der ungeladenen Nebenprodukte des Ionisationsprozesses sind der Stutzen 23 und der untere Abschnitt des Außengewindes 22 mit Leitungen 24 und 25 versehen.

Während die Leitung 25 mit der Saugseite eines (nicht dargestellten) Miniatur-Gebläses verbunden ist, wird der Ionisationselektrode 4' über die Leitung 24 das zu ionisierende Gas zugeführt. Dabei kann es sich um Umgebungsluft oder um medizinischen Sauerstoff handeln, wobei letzterer beispielsweise einer Druckflasche entnommen werden kann.

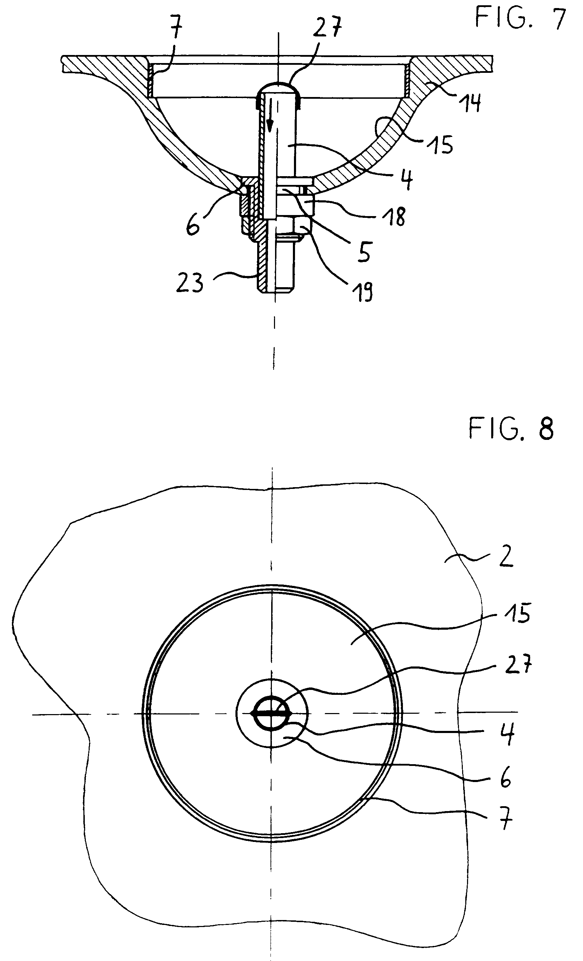

In den Figuren 7 und 8 ist ein weiteres Ausführungsbeispiel gezeigt, bei dem die ungeladenen Nebenprodukte des Ionisationsprozesses unmittelbar über die Ionisationselektrode 4 abgesaugt werden. Hierzu wird an dem hohlzylindrischen Elektrodenhalter 5 eine mit einem Miniatur-Gebläse verbundene Absaugleitung angeschlossen. Eine besondere Zuführung der zu ionisierenden Umgebungsluft, etwa durch ein zusätzliches Miniatur-Gebläse, ist hier nicht vorgesehen.

Die selektive Absaugung der Nebenprodukte wird durch den Umstand begünstigt, daß die negativ geladenen Sauerstoffanionenradikale im Gegensatz zu den ungeladenen Nebenprodukten entlang der Trajektorien zum Punkt des niedrigsten Potential driften und von der Saugströmung nahezu unbeeinflußt bleiben. Das elektrische Feld, welches durch die besondere Ausgestaltung und Anordnung der Elektroden (Ionisationselektrode und Gegenelektrode) sowie die Auslegung der elektrischen Parameter bestimmt ist, trägt hier somit wesentlich zu der Abtrennung der ungeladenen Nebenprodukte bei. Diese werden in größerem Abstand zum Patienten ins Freie geführt. Auch kann gegebenenfalls eine chemischen Weiterbehandlung vorgsehen werden.

Die Erfindung beschränkt sich in ihrer Ausführung nicht auf die vorstehend angegebenen Ausführungsbeispiele. Vielmehr ist eine Anzahl von Varianten denkbar, die von der dargestellten Lösung auch bei grundsätzlich anders gearteten Ausführungen Gebrauch machen.

### Bezugszeichenliste

- 1: Inhalationsgerät
- 2: Gehäuse
- 3: Abstandhalter
- 4, 4': Ionisationselektrode
- 5, 5': Elektrodenhalter
- 6, 6': Flansch
- 7, 7': Gegenelektrode
- 8: elektronische Schaltung
- 11: Sauggebläse
- 12: Ringleitung
- 13, 13': Absaugöffnungen
- 14, 14': Reflektor
- 15: Innenseite des Reflektors
- 16: Bohrung im Reflektor
- 18: Kontaktscheibe
- 19, 19': Mutter
- 20: Saugkappe
- 21: Stutzen
- 22: Außengewinde
- 23: Stutzen
- 24: Leitung
- 25: Leitung
- 26: Display
- 27: Kohlenstoffasern

## Patentansprüche

1. Verfahren zur Erzeugung von gasförmigen Sauerstoffanionenradikalen unter atmosphärischen Druckbedingungen und Verwendung einer aus Kohlenstoffasern bestehenden Ionisationselektrode, dadurch gekennzeichnet, daß ungeladene Nebenprodukte des Ionisationsprozesses, wie Stickoxide und Ozon, von den erzeugten Sauerstoffanionenradikalen durch Absaugen abgetrennt werden.

2. Verfahren nach Anspruch 1, dadurch gekennzeichnet, daß im Bereich der Ionisationselektrode (4) eine gerichtete Saugströmung einstellt wird, deren Saugwirkung im wesentlichen auf den engeren Bereich der Ionisationselektrode (4) begrenzt ist und die erzeugten Sauerstoffanionenradikale an einem davon beabstandeten Applikationsort im wesentlichen nicht erfaßt.

3. Verfahren nach Anspruch 1 oder 2, dadurch gekennzeichnet, daß eine Gegenelektrode (7) verwendet wird, die den gesamten Bereich der Ionisationselektrode (4) beeinflußt und den Aufbau eines hinsichtlich der Geometrie und der Spannung definierten elektrischen Feldes ermöglicht.

4. Verfahren nach Anspruch 3, dadurch gekennzeichnet, daß die Gegenelektrode (7) hochohmig an das Bezugspotential einer Hochspannungsquelle angekoppelt ist und ein Potentialunterschied mit U_{Bezugspotential} < U_{Gegenelektrode} < U_{Ionisationselektrode} erzeugt wird.

5. Verfahren nach Anspruch 3, dadurch gekennzeichnet, daß die Gegenelektrode (7) an einer Spannung kleiner 7 KV angeschlossen ist.

6. Verfahren nach einem der Ansprüche 3 bis 5, dadurch gekennzeichnet, daß im Bereich der Ionisationselektrode (4) ein elektrisches Feld zwischen 30 KV/m und 1500 KV/m eingestellt wird.

7. Verfahren nach einem der vorstehenden Ansprüche, dadurch gekennzeichnet, daß das zu ionisierende Gas, die Ionisationselektrode (4) und/oder die Umgebung der Ionisationselektrode (4) beheizt werden.

8. Verfahren nach einem der vorstehenden Ansprüche, dadurch gekennzeichnet, daß der Ionisationselektrode (4) das zu ionisierende Gas derart zugeführt wird, daß eine Innenbelüftung der Ionisationselektrode erfolgt.

9. Vorrichtung zur Erzeugung von Sauerstoffanionenradikalen unter atmosphärischen Druckbedingungen mit einer aus Kohlenstoffasern bestehenden Ionisationselektrode, gekennzeichnet durch eine Absaugeinrichtung, mittels der ungeladene Nebenprodukte des Ionisationsprozesses von den erzeugten Sauerstoffanionenradikalen abtrennbar sind.

10. Vorrichtung nach Anspruch 9, dadurch gekennzeichnet, daß, die Absaugöffnungen (13) der Absaugeinrichtung (11, 12) in unmittelbarer Umgebung der Ionisationselektrode (4) angeordnet sind.

11. Vorrichtung nach Anspruch 10, dadurch gekennzeichnet, daß, die Absaugöffnungen (13') in einem Elektrodenhalter (5') ausgebildet sind.

12. Vorrichtung nach Anspruch 9 oder 10, dadurch gekennzeichnet, daß die Ionisationselektrode (4) zentrisch in einem Reflektor (14) angeordnet ist, welcher eine oder mehrere Absaugöffnungen (13) aufweist, die so angeordnet sind, daß sich im Bereich der Ionisationselektrode (4) eine Saugströmung einstellt, deren Saugwirkung im wesentlichen auf den engeren Bereich der Ionisationselektrode (4) begrenzt ist und die erzeugten Sauerstoffanionenradikale an einem davon beabstandeten Applikationsort im wesentlichen nicht erfaßt.

13. Vorrichtung nach Anspruch 12, dadurch gekennzeichnet, daß der Reflektor (14) kalottenförmig ausgebildet ist und eine ringförmige Gegenelektrode (7) aufweist, die hochohmig an das Massepotential einer Hochspannungsquelle angekoppelt ist, so daß ein Potentialunterschied mit U_{Bezugspotential} < U_{Gegenelektrode} < U_{Ionisationselektrode} vorhanden ist.

14. Vorrichtung nach einem der Ansprüche 9 bis 13, dadurch gekennzeichnet, daß die Ionisationselektrode (4) an eine negative Spannung von 1 bis 7 KV zum Bezugspotential einer Hochspannungsquelle angeschlossen ist.

15. Vorrichtung nach einem der Ansprüche 9 bis 12, dadurch gekennzeichnet, daß eine Gegenelektrode (7) vorgesehen ist, die an einer Spannung kleiner 7 KV angeschlossen ist.

16. Vorrichtung nach einem der Ansprüche 9 bis 15, dadurch gekennzeichnet, daß im Bereich der Ionisationselektrode (4) ein elektrisches Feld zwischen 30 KV/m und 1500 KV/m einstellbar ist.

17. Vorrichtung nach einem der Ansprüche 9 bis 16, dadurch gekennzeichnet, daß eine Heizeinrichtung vorgesehen ist, mit der das zu ionisierende Gas, die Ionisationelektrode (4) und/oder der kalottenförmige Reflektor (14) beheizbar sind.

18. Vorrichtung nach einem der Ansprüche 9 bis 17, dadurch gekennzeichnet, daß die Ionisationselektrode (4) hohlzylindrisch ausgebildet und mit einer Leitung verbundenen ist, über welche die ungeladenen Nebenprodukte des Ionisationsprozesses abgesaugt werden.

19. Vorrichtung nach einem der Ansprüche 9 bis 17, dadurch gekennzeichnet, daß die Ionisationselektrode (4) mit einer Leitung (24) verbunden ist, über die ihr das zu ionisierende Gas zugeführt wird.

20. Vorrichtung nach Anspruch 19, dadurch gekennzeichnet, daß das zu ionisierende Gas Umgebungsluft ist, die über ein Gebläse angesaugt wird.

## Claims

1. A method for producing gaseous oxygen anion radicals under atmospheric pressure using an ionization electrode consisting of carbon fibres, characterized in that uncharged by-products of the ionization process such as nitrogen oxides or ozone are separated by suction from the oxygen anion radicals produced.

2. Method according to claim 1, characterized in that a suction draft is directed to the area around the ionization electrode (4) so that the suction effect is basically limited to the closer area around the ionization electrode (4) and does not affect the oxygen anion radicals produced to any appreciable extent at their somewhat more distant place of application.

3. Method according to claims 1 or 2, characterized in that a counter-electrode (7) is used that influences the whole range of the ionization electrode (4) so that an electric field with a defined geometry and voltage can be built up.

4. Method according to claim 3, characterized in that the counter-electrode(7) is connected to the reference potential of a high voltage source via a high-resistance coupling, and that a potential difference is created where U_{reference potential} < U_{counter-electrode} < U_{ionization electrode}.

5. Method according to claim 3, characterized in that the counter-electrode(7) is connected to a negative voltage smaller than -7 kV.

6. Method to any one of claims 3 to 5, characterized in that an electric field with a strength between 30 kV/m and 1500 kV/m is built up in the range of the ionization electrode (4).

7. Method according to any one of the above-mentioned claims, characterized in that the gas to be ionized, the ionization electrode (4) and/or the environment of the ionization electrode (4) are heated.

8. Method according to any one of the above-mentioned claims, characterized in that the gas to be ionized is fed to the ionization electrode (4) in such a way that said ionization electrode is internally ventilated.

9. An apparatus for producing gaseous oxygen anion radicals under atmospheric pressure using an ionization electrode consisting of carbon fibres, characterized in that it comprises a suction device by which uncharged by-products of the ionization process can be separated from the oxygen anion radicals produced.

10. Apparatus according to claim 9, characterized in that the suction holes (13) of said suction device (11, 12) are placed in the immediate vicinity of the ionization electrode (4).

11. Apparatus according to claim 10, characterized in that said suction holes (13') are placed in an electrode holder (5').

12. Apparatus accoring to claims 9 or 10, characterized in that the ionization electrode (4) is centered in a reflector (14) that comprises one or several suction holes (13), said suction holes being arranged in such a way that a suction draft occurs in the area around the ionization electrode (4), the suction effect of which is limited to the closer surroundings of the ionization electrode (4) and does not affect the oxygen anion radicals produced to any appreciable extent at their somewhat distant place of application.

13. Apparatus according to claim 12, characterized in that the reflector (14) is cap-shaped and comprises a ring-shaped counter-electrode (7) that is connected to the reference potential of a high voltage source via a high-resistance coupling, so that a potential difference is created where U_{reference potential} < U_{counter-electrode} < U_{ionization electrode}.

14. Apparatus according to any one of claims 9 to 13, characterized in that the ionization electrode (4) is connected to a negative voltage of -1 to -7 kV to the reference potential of a high voltage source.

15. Apparatus according to any one of claims 9 to 12, characterized in that it is equipped with a counter-electrode (7) that is connected to a negative voltage smaller than -7 kV.

16. Apparatus according to any one of claims 9 to 15, characterized in that an electric field with a strength between 30 kV/m and 1500 kV/m can be built up in the area surrounding the ionization electrode (4).

17. Apparatus according to any one of claims 9 bis 16, characterized in that it is equipped with a heating device with which the gas to be ionized, the ionization electrode (4) and/or the cap-shaped reflector (14) can be heated.

18. Apparatus according to any one of claims 9 bis 17, characterized in that the ionization electrode (4) has a tubular shape and is connected to a line via which the uncharged by-products of the ionization process are separated by suction.

19. Apparatus according to any one of claims 9 bis 17, characterized in that the ionization electrode (4) is connected with a line (24) via which the gas to be ionized is fed to it.

20. Apparatus according to claim 19, characterized in that the gas to be ionized is ambient air that is drawn in via an aspirator unit.

## Revendications

1. Procédé de production de radicaux anioniques d'oxygène gazeux dans des conditions de pression atmosphériques et utilisation d'une électrode d'ionisation constituée de fibres de carbone,
caractérisé en ce qu'
on sépare des sous produits non chargés du processus d'ionisation, comme des oxydes de l'azote et de l'ozone des radicaux anioniques d'oxygène par aspiration.

2. Procédé selon la revendication 1,
caractérisé en ce que
dans le domaine de l'électrode d'ionisation (4) on ajuste un courant dirigé d'aspiration, dont l'effet d'aspiration est limité essentiellement au domaine contigu à l'électrode d'ionisation (4) et ne concerne pratiquement pas les radicaux anioniques d'oxygène produits en un lieu d'application qui en est éloigné.

3. Procédé selon la revendication 1 ou 2,
caractérisé en ce qu'
on utilise une contre-électrode (7) qui influe tout le domaine de l'électrode d'ionisation (4) et permet la constitution d'un champ électrique défini du point de vue de la géométrie et de la tension.

4. Procédé selon la revendication 3,
caractérisé en ce que
la contre-électrode (7) est couplée à l'aide d'une résistance élevée au potentiel de référence d'une source à haute tension et qu'on produit une différence de potentiel telle que U_{potentiel de référence} < U_{contre-électrode} < U_{électrode d'ionisation}.

5. Procédé selon la revendication 3,
caractérisé en ce que
la contre-électrode (7) est branchée à une tension inférieure à 7 KV.

6. Procédé selon l'une des revendications 3 à 5,
caractérisé en ce que
dans le domaine de l'électrode d'ionisation (4), on établit un champ électrique compris entre 30 KV/m et 1500 KV/m.

7. Procédé selon l'une des revendications précédentes,
caractérisé en ce qu'
on chauffe le gaz à ioniser, l'électrode d'ionisation (4) et/ou l'environnement de l'électrode d'ionisation (4).

8. Procédé selon l'une des revendications précédentes,
caractérisé en ce qu'
on introduit le gaz à ioniser au niveau de l'électrode d'ionisation (4) de façon à réaliser une ventilation intérieure de l'électrode d'ionisation.

9. Dispositif de production de radicaux anioniques d'oxygène dans des conditions de pression atmosphériques avec une électrode d'ionisation constituée de fibres de carbone,
caractérisé
par un dispositif d'aspiration grâce auquel les sous produits non chargés du processus d'ionisation sont séparables des radicaux anioniques d'oxygène produits.

10. Dispositif selon la revendication 9,
caractérisé en ce que
les orifices d'aspiration (13) du dispositif d'aspiration (11, 12) sont disposés au voisinage immédiat de l'électrode d'ionisation (4).

11. Dispositif selon la revendication 10,
caractérisé en ce qu'
on forme les orifices d'aspiration (13') dans un support d'électrode (5').

12. Dispositif selon la revendication 9 ou 10,
caractérisé en ce que
l'électrode d'ionisation (4) est disposée centralement dans un réflecteur (14) qui présente un ou plusieurs orifices d'aspiration (13) qui sont implantés de telle sorte que dans le domaine de l'électrode d'ionisation (4), il s'établit un écoulement d'aspiration, dont l'effet d'aspiration est essentiellement limité au domaine contigu à l'électrode d'ionisation (4) et ne concerne pratiquement pas les radicaux anioniques d'oxygène produits en un lieu d'application qui en est éloigné.

13. Dispositif selon la revendication 12,
caractérisé en ce que
le réflecteur (14) est constitué en forme de calotte et présente une contre-électrode (7) annulaire, qui est couplée par l'intermédiaire d'une résistance élevée au potentiel de masse d'une source à haute tension, de sorte qu'il existe une différence de potentiel telle que U_{potentiel de référence} < U_{contre-électrode} < U_{électrode d'ionisation}.

14. Dispositif selon l'une des revendications 9 à 13,
caractérisé en ce que
l'électrode d'ionisation (4) est branchée à une tension négative de 1 à 7 KV par rapport au potentiel de référence d'une source à haute tension.

15. Dispositif selon l'une des revendications 9 à 12,
caractérisé en ce qu'
on prévoit une contre-électrode (7) qui est branchée à une tension inférieure à 7 KV.

16. Dispositif selon l'une des revendications 9 à 15,
caractérisé en ce que
dans le domaine de l'électrode d'ionisation (4), on peut établir un champ électrique entre 30 KV/m et 1500 KV/m.

17. Dispositif selon l'une des revendications 9 à 16,
caractérisé en ce qu'
on prévoit un dispositif de chauffage, grâce auquel on peut chauffer le gaz à ioniser, l'électrode d'ionisation (4) et/ou le réflecteur en forme de calotte (14).

18. Dispositif selon l'une des revendications 9 à 17,
caractérisé en ce que
l'électrode d'ionisation (4) est en forme de cylindre creux et est reliée à une conduite, par où on peut aspirer les sous produits non chargés du processus d'ionisation.

19. Dispositif selon l'une des revendications 9 à 17,
caractérisé en ce que
l'électrode d'ionisation (4) est reliée à une conduite (24) par laquelle on introduit le gaz à ioniser.

20. Dispositif selon la revendication 19,
caractérisé en ce
le gaz à ioniser est de l'air environnant, qui est aspiré par un ventilateur.
